(19) Europäisches Patentamt / European Patent Office / Office européen des brevets

(11) **EP 2 887 902 B1**

(12) **FASCICULE DE BREVET EUROPEEN**

(45) Date de publication et mention
de la délivrance du brevet:
**22.03.2017 Bulletin 2017/12**

(21) Numéro de dépôt: **13756363.1**

(22) Date de dépôt: **22.08.2013**

(51) Int Cl.:
*A61B 5/00* (2006.01)     *A61B 90/30* (2016.01)
*F21V 8/00* (2006.01)     *G02B 6/32* (2006.01)
*G02B 6/04* (2006.01)     *G02B 6/26* (2006.01)

(86) Numéro de dépôt international:
**PCT/EP2013/067446**

(87) Numéro de publication internationale:
**WO 2014/029838 (27.02.2014 Gazette 2014/09)**

(54) **DISPOSITIF D'ECLAIRAGE DOUBLE FLUX MULTIFIBRES OPTIQUES ET SONDE PEROPERATOIRE ASSOCIEE**

BELEUCHTUNGSVORRICHTUNG MIT DOPPELTEM LICHTSTROM UND MEHREREN OPTISCHEN FASERN UND ZUGEHÖRIGE PEROPERATIVE SONDE

DOUBLE-FLUX LIGHTING DEVICE INCLUDING MULTIPLE OPTICAL FIBRES, AND ASSOCIATED PEROPERATIVE PROBE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.08.2012 FR 1257974**

(43) Date de publication de la demande:
**01.07.2015 Bulletin 2015/27**

(73) Titulaire: **Commissariat à l'Energie Atomique et aux Energies Alternatives
75015 Paris (FR)**

(72) Inventeurs:
• **COUTARD, Jean-Guillaume**
 **F-38100 Grenoble (FR)**

• **BERGER, Michel**
 **F-38640 Claix (FR)**
• **GIOUX, Sylvain**
 **F-33670 Créon (FR)**

(74) Mandataire: **Bréda, Jean-Marc et al
Marks & Clerk France
Conseils en Propriété Industrielle
Immeuble " Visium "
22 Avenue Aristide Briand
94117 Arcueil Cedex (FR)**

(56) Documents cités:
**WO-A1-95/12090      US-A- 4 964 692
US-A1- 2004 156 098**

**EP 2 887 902 B1**

## Description

Le domaine de l'invention est celui des dispositifs d'éclairage comportant un transport de lumière comportant une pluralité de fibres optiques multimodes. Ce transport est également connu sous le terme anglais de « bundle ».

Ces transports optiques ont de multiples applications, notamment dans le domaine de l'optique instrumentale pour les applications biomédicales. Ces applications nécessitent des éclairages ayant des caractéristiques particulières. On citera en particulier les applications destinées à la chirurgie oncologique guidée par fluorescence.

Basée sur la capacité à exciter puis à collecter la fluorescence d'un traceur préalablement injecté au patient, la chirurgie oncologique réclame, outre le développement de traceurs spécifiques aux cancers, la conception de sondes peropératoires destinées à guider le chirurgien.

Ces sondes doivent posséder des caractéristiques très spécifiques. Ces instruments doivent délivrer sur les tissus une lumière d'excitation du fluorophore compatible avec une bonne sensibilité de détection ainsi qu'une lumière blanche puissante afin que le praticien ait une bonne vision des tissus visualisés. De plus, certaines applications médicales nécessitent une taille réduite de la tête optique. A titre d'exemple, le diamètre de la tête optique d'une sonde peropératoire ne doit pas excéder quelques dizaines de millimètres, typiquement entre 15 et 50 millimètres.

Compte tenu de l'utilisation finale de cet éclairage, l'innocuité de la lumière délivrée pour les tissus vivants et les yeux est également un impératif. A flux optique délivré donné, il est donc nécessaire de multiplier les points sources délivrés par le bundle de fibres optiques afin d'en diminuer leur puissance et donc leur dangerosité. L'objectif est qu'en sortie de sonde, l'éclairage appartienne à la classe « laser 1 » qui est la classe d'énergie la plus faible, selon le décret français N° 2010-750 du 2 juillet 2010 relatif à la protection des travailleurs contre les risques dus aux rayonnements optiques artificiels et selon la directive européenne N° 2006/25/CE du 5 avril 2006 relative aux prescriptions minimales de sécurité et de santé relatives à l'exposition des travailleurs aux risques dus aux agents physiques (rayonnements optiques artificiels).

Plusieurs dispositifs d'éclairage à fibres optiques ont été proposés. On citera notamment le dispositif d'éclairage décrit dans le brevet US 4 272 156 qui propose de tailler les faces de sortie des fibres optiques selon des profils et des inclinaisons particulières pour améliorer l'homogénéité de l'éclairage. On citera également le dispositif d'éclairage décrit dans le brevet US 5 412 749 permettant de mélanger dans une fibre optique unique la lumière issue de deux sources différentes. Enfin, on citera le dispositif décrit dans le brevet US 4 964 692 qui décrit un système optique disposé en sortie de « bundle » et qui permet d'obtenir un éclairage homogène.

La demande de brevet WO 98/05987 décrit un dispositif d'éclairage homogène d'un bundle de fibre. La demande de brevet US 2004/0156098 A1 décrit un système d'éclairage d'une ouverture comprenant deux lentilles.

Ces dispositifs ne permettent pas de prendre en compte l'ensemble des caractéristiques et des contraintes d'une sonde peropératoire telles qu'elles ont été définies ci-dessus. Le dispositif d'éclairage double flux multifibres optiques selon l'invention remplit cet objectif. Il comporte des moyens optiques agencés de façon à obtenir à l'entrée du bundle un éclairage de même intensité et de même ouverture sur chaque fibre de façon à obtenir en sortie un éclairage plus homogène. Par éclairage plus homogène, on entend que la puissance lumineuse délivrée par chaque fibre est faiblement dispersée. D'autre part, cette méthode permet d'optimiser le flux total donné par l'éclairage sans jamais dépasser les niveaux de sécurité autorisés sur chaque fibre optique. En effet, lorsque l'éclairage est inhomogène, il suffit qu'une seule fibre optique soit au-dessus du seuil autorisé pour que le dispositif d'éclairage ne réponde pas aux normes de sécurité oculaire. Au contraire, avec l'invention, du fait de l'homogénéité de la puissance lumineuse délivrée par chaque fibre, la puissance lumineuse de chaque fibre peut être proche de la puissance lumineuse maximale autorisée. L'éclairage est donc plus homogène et plus intense, tout en respectant les normes de sécurité.

Plus précisément, l'invention a pour objet un dispositif d'éclairage comprenant au moins une source de lumière fibrée et un transport de lumière comportant une pluralité de secondes fibres optiques multimodes, la lumière issue de la source de lumière étant conduite à une extrémité de sortie d'une première fibre optique, les premières extrémités des secondes fibres optiques étant regroupées généralement de façon sensiblement homogène dans une première surface, les secondes extrémités des fibres optiques étant disposées sur la périphérie d'une seconde surface, caractérisé en ce que le dispositif d'éclairage comporte :

une première lentille disposée entre l'extrémité de sortie de la première fibre optique et les premières extrémités des secondes fibres optiques, ladite extrémité de sortie de la première fibre optique étant disposée sensiblement sur l'axe optique et au foyer principal objet de ladite première lentille ; et lesdites premières extrémités des secondes fibres optiques étant disposées sensiblement sur l'axe optique et au foyer principal image de ladite première lentille ; un diffuseur optique ayant la forme d'une lame, en particulier une lame mince, disposé entre la première lentille et les premières extrémités des secondes fibres optiques.

Avantageusement, la focale de la première lentille est sensiblement égale au quotient du rayon de la première surface circulaire par l'ouverture numérique de la première fibre optique et l'angle de diffusion du diffuseur optique est sensiblement égal à l'ouverture numérique des secondes fibres optiques.

La première surface peut être circulaire. La deuxième surface est de préférence annulaire, ce qui permet une bonne adaptation autour d'une caméra de forme cylindrique. De préférence, les secondes extrémités des fibres optiques sont disposées de façon sensiblement équidistante sur la périphérie de ladite deuxième surface.

L'invention concerne également un dispositif d'éclairage comprenant au moins une source de lumière fibrée et un transport de lumière comportant une pluralité de secondes fibres optiques multimodes, la lumière issue de la source de lumière étant conduite à une extrémité de sortie d'une première fibre optique, les premières extrémités des secondes fibres optiques étant regroupées généralement de façon sensiblement homogène dans une première surface, les secondes extrémités des fibres optiques étant disposées sur la périphérie d'une seconde surface, caractérisé en ce que le dispositif d'éclairage comporte :

Un système afocal optique comprenant une première lentille et une seconde lentille, ledit système disposé entre l'extrémité de sortie de la première fibre optique et les premières extrémités des secondes fibres optiques, le foyer principal image de la première lentille étant confondu avec le foyer principal objet de la seconde lentille ;

L'extrémité de sortie de la première fibre optique étant disposée sensiblement sur l'axe optique et au foyer principal objet de ladite première lentille ; et lesdites premières extrémités des secondes fibres optiques étant disposées sensiblement sur l'axe optique et au foyer principal image de ladite seconde lentille ;

un diffuseur optique ayant la forme d'une lame disposé au voisinage du foyer principal image de la première lentille et au foyer principal objet de la seconde lentille.

La première surface peut être circulaire. La deuxième surface est de préférence annulaire, ce qui permet une bonne adaptation autour d'une caméra de forme cylindrique. De préférence, les secondes extrémités des fibres optiques sont disposées de façon sensiblement équidistante sur la périphérie de ladite deuxième surface.

Le système afocal peut avoir un grandissement unitaire et une ouverture numérique sensiblement égale à l'ouverture numérique des secondes fibres optiques.

D'une façon générale, l'invention concerne un dispositif d'éclairage comprenant au moins une source de lumière fibrée et un transport de lumière comportant une pluralité de secondes fibres optiques multimodes, la lumière issue de la source de lumière étant conduite à une extrémité de sortie d'une première fibre optique, les premières extrémités des secondes fibres optiques étant regroupées généralement de façon sensiblement homogène dans une première surface, les secondes extrémités des fibres optiques étant disposées sur la périphérie d'une seconde surface, caractérisé en ce que le dispositif d'éclairage comporte :

- Une lentille, disposée entre l'extrémité de sortie de la première fibre optique et les premières extrémités des secondes fibres optiques, de telle sorte que lesdites premières extrémités des secondes fibres optiques sont disposées sensiblement au foyer principal image de ladite lentille ;
- Un diffuseur optique, sous la forme d'une lame constituée par un matériau diffusant, disposé entre l'extrémité de sortie de la première fibre optique et les premières extrémités des secondes fibres optiques.

Selon un mode de réalisation, le dispositif comporte une première lentille disposée entre l'extrémité de sortie de la première fibre optique et les premières extrémités des secondes fibres optiques, ladite extrémité de sortie de la première fibre optique étant disposée sensiblement sur l'axe optique et au foyer principal objet de ladite première lentille, et lesdites premières extrémités des secondes fibres optiques étant disposées sensiblement sur l'axe optique et au foyer principal image de ladite première lentille. Selon ce mode de réalisation, le diffuseur est de préférence disposé au voisinage du foyer principal image de la première lentille.

Selon un autre mode de réalisation, le dispositif comporte une première lentille et une seconde lentille, le foyer principal image de la première lentille étant confondu avec le foyer principal objet de la seconde lentille, l'extrémité de sortie de la première fibre optique étant alors disposée sensiblement sur l'axe optique et au foyer principal objet de ladite première lentille, et lesdites premières extrémités des secondes fibres optiques étant disposées sensiblement au foyer principal image de ladite seconde lentille. Selon ce mode de réalisation, le diffuseur est de préférence disposé au voisinage du foyer principal image de la première lentille et au foyer principal objet de la seconde lentille.

Avantageusement, le dispositif d'éclairage comporte une seconde source de lumière et un coupleur optique comprenant deux entrées et une sortie, la seconde source ayant un second spectre d'émission différent du premier spectre d'émission de la première source, la première source de lumière étant disposée devant la première entrée du coupleur, la seconde source de lumière étant disposée devant la seconde entrée du coupleur, la sortie du coupleur étant disposée au foyer principal objet de la première lentille.

Avantageusement, le dispositif d'éclairage comporte une seconde source de lumière fibrée et une lame semi-réfléchissante,

la seconde source ayant un second spectre d'émission différent du premier spectre d'émission de la première source, la lumière issue de la seconde source de lumière étant conduite à une extrémité de sortie d'une troisième fibre optique,

la lame semi-réfléchissante comportant un traitement dichroïque optimisé de façon à avoir un maximum de transmission du premier spectre d'émission et un maximum de réflexion du second spectre d'émission, la lame semi-réfléchissante étant disposée de façon que l'image de l'extrémité de sortie de la troisième fibre optique par réflexion sur la lame semi-réfléchissante se superpose à l'image par transmission de l'extrémité de sortie de la première fibre optique.

L'invention concerne également une sonde peropératoire comprenant une caméra et un dispositif d'éclairage comme défini ci-dessus, le premier spectre de la première source étant situé dans le visible, le second spectre de la seconde source étant situé dans un spectre de fluorescence situé dans le proche infrarouge, les secondes extrémités des fibres optiques étant disposées de façon sensiblement équidistante sur la périphérie de l'objectif de la caméra.

L'invention sera mieux comprise et d'autres avantages apparaîtront à la lecture de la description qui va suivre donnée à titre non limitatif et grâce aux figures annexées parmi lesquelles :

La figure 1 représente le schéma général d'un dispositif d'éclairage selon l'invention ;
Les figures 2 et 3 représentent une vue du transport de lumière d'un dispositif d'éclairage selon l'invention ;
La figure 4 représente un premier mode de réalisation des moyens optiques de conjugaison entre la source de lumière et le transport de lumière ;
Les figures 5 et 6 représentent un second mode de réalisation des moyens optiques de conjugaison entre la source de lumière et le transport de lumière ;
La figure 7 représente un premier moyen de couplage de deux sources de lumière différentes ;
La figure 8 représente un second moyen de couplage de deux sources de lumière différentes.

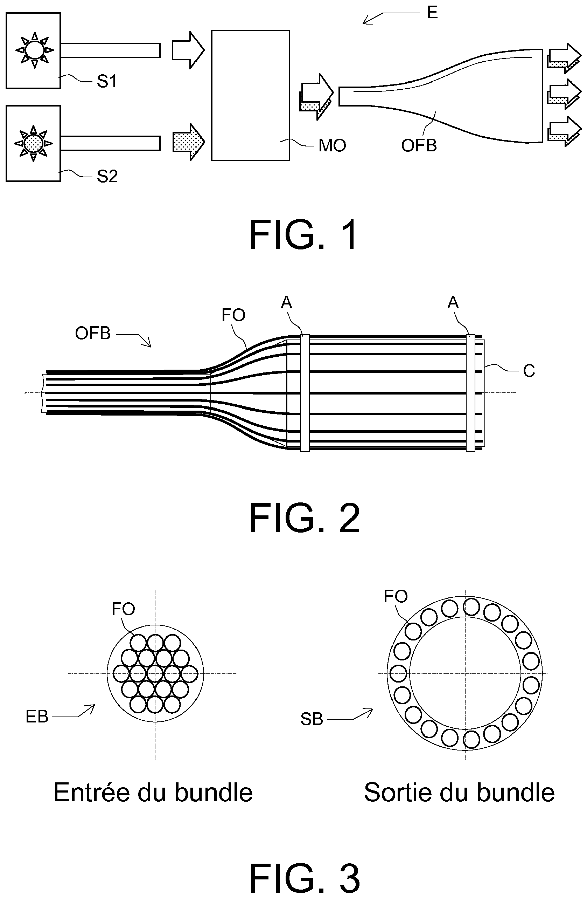

A titre d'exemple, la figure 1 représente le schéma général d'un dispositif d'éclairage E selon l'invention. Sur cette figure, il comporte deux sources d'éclairage différentes S1 et S2 fibrées, des moyens optiques MO agencés de façon à conjuguer la ou les fibres optiques d'éclairage avec l'entrée d'un transport OFB de lumière à fibres optiques multimodes FO ou « optical fiber bundle ». La fonction du transport de lumière à fibres optiques est double. D'une part, il permet de véhiculer la lumière et d'autre part, il permet de la répartir différemment. Les figures 2 et 3 illustrent ces fonctions. La figure 2 représente la sortie des fibres optiques FO multimodes du bundle. Elles sont réparties de façon homogène autour de la tête optique d'une caméra C qu'elles enveloppent. Elles sont maintenues autour de la tête optique par deux anneaux A de maintien. La figure 3 représente une vue de face de l'entrée EB et une vue de face de la sortie SB du bundle. A l'entrée EB du bundle, les extrémités des fibres optiques FO sont regroupées dans un hexagone inscrit dans une première surface circulaire. A la sortie SB du bundle, les extrémités des fibres optiques sont disposées de façon sensiblement équidistante sur la périphérie d'une seconde surface circulaire. A titre indicatif, dix-neuf fibres optiques multimodes sont représentées sur la figure 3.

Ce type d'éclairage est utilisé notamment dans des sondes optiques peropératoires. Généralement, comme indiqué sur la figure 1, le dispositif d'éclairage de ce type de sonde comporte deux sources. La première est destinée à assurer un éclairage dans le spectre visible, la seconde source qui est généralement une source laser émet dans le proche infrarouge et est destinée à provoquer un rayonnement de fluorescence des tissus vivants. La longueur d'onde d'émission peut être située autour de 740 nanomètres.

Une des difficultés de ce type d'éclairage est la transmission de la lumière issue de la source de lumière fibrée à l'entrée des fibres optiques du bundle optique. En effet, cette transmission de lumière doit se faire avec un minimum de pertes. Chaque fibre du bundle doit recevoir sensiblement le même flux. Enfin, les faisceaux de lumière entrant dans chaque fibre doivent avoir la même ouverture numérique correspondant sensiblement à celle des fibres optiques du bundle. Dans ce qui suit, l'ouverture numérique des fibres correspond au cône d'acceptance des fibres optiques défini de façon classique par la relation suivante : $\sin(i) = \sqrt{n_1^2 - n_2^2}$ i étant le demi-angle d'acceptance, $n_1$ étant l'indice optique du coeur de la fibre optique et $n_2$ étant l'indice optique de la gaine de la fibre optique.

De façon à répondre au mieux à ces exigences, le dispositif d'éclairage selon l'invention comprend des moyens de conjugaison optique. Ces moyens comportent une ou deux lentilles et un diffuseur optique.

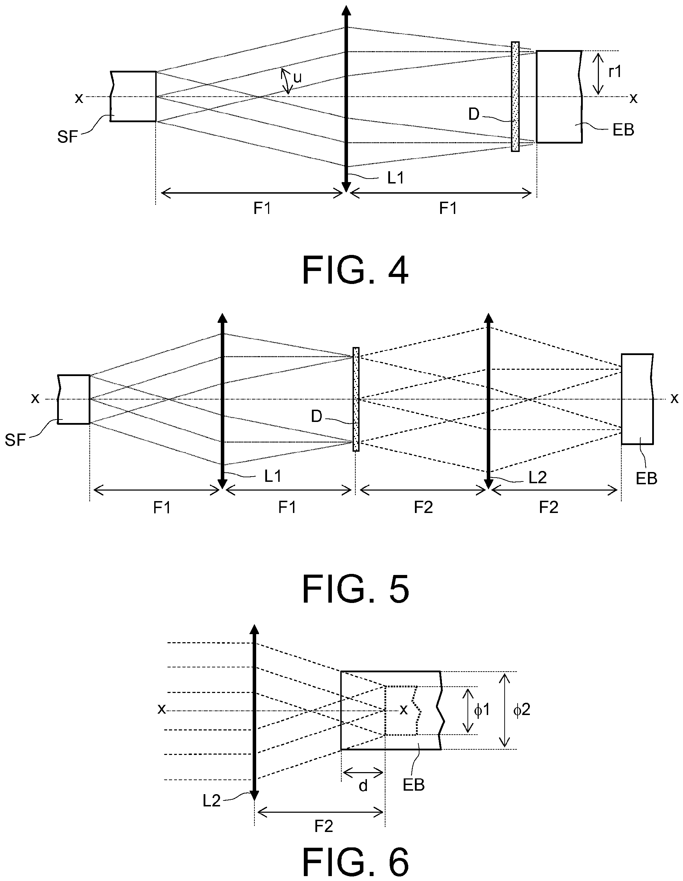

Les figures 4, 5, 6 et 8 représentent les moyens de conjugaison selon l'invention. Sur ces différentes figures, les lentilles optiques sont représentées conventionnellement par des doubles flèches. Ces lentilles peuvent être des lentilles minces ou des moyens optiques assimilables à des lentilles minces comme les lentilles à gradients d'indice, par exemple. Les rayons lumineux sont représentés par des traits en pointillés.

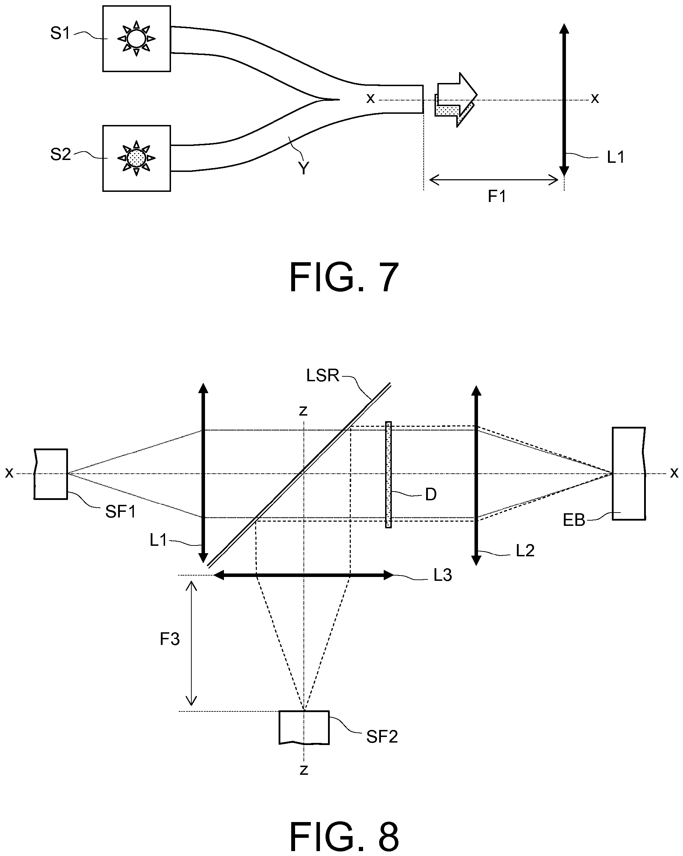

La figure 4 représente un premier mode de réalisation de ces moyens de conjugaison. Sur cette vue, seule une sortie de source fibrée SF est représentée. Comme on le verra sur la figure 8, il est facile d'adapter ce dispositif à la mise en oeuvre de deux sources fibrées. Les moyens de conjugaison comportent une première lentille L1 disposée entre l'extrémité de sortie SF de la source fibrée et les extrémités des fibres optiques de l'entrée du bundle EB, l'extrémité de sortie SF de la source fibrée étant disposée sensiblement sur l'axe optique xx et au foyer principal objet de ladite première lentille L1 et les premières extrémités des fibres optiques de l'entrée du bundle étant disposées sensiblement sur l'axe optique xx et au foyer principal image de la première lentille L1. Un diffuseur optique D ayant la forme d'une lame mince est disposé entre la première lentille et les extrémités des fibres optiques du bundle. Le terme mince désigne le fait que son épaisseur est typiquement comprise entre 1 et 20 mm. Le fait de placer les extrémités des fibres optiques de l'entrée du bundle EB à la distance focale de la première lentille L1 permet d'optimiser l'efficacité du couplage optique. On comprend, sur la figure 4 , qu'en plaçant l'entrée du bundle EB à une autre distance, inférieure ou supérieure à la distance focale, on réduit la quantité de lumière collectée par le bundle.

Comme indiqué sur la figure 3, les extrémités des fibres optiques du bundle sont regroupées de façon sensiblement homogène dans une première surface circulaire. De façon préférentielle, la focale F1 de la première lentille L1 est sensiblement égale au quotient du rayon r1 de la première surface circulaire par l'ouverture numérique u de la fibre optique de la source fibrée SF et l'angle de diffusion $\alpha$ du diffuseur optique D est sensiblement égal à l'ouverture numérique u1 des fibres optiques du bundle.

Comme on le voit sur la figure 4, l'ensemble de ces dispositions présente les avantages suivants. Chaque point de la source fibrée éclaire la totalité de la première surface circulaire, l'éclairement étant ainsi homogène sur toutes les fibres optiques de l'entrée du bundle. Chaque fibre optique du bundle reçoit cet éclairement dans un angle d'ouverture identique. Cet angle d'ouverture correspond parfaitement, grâce au diffuseur, à l'ouverture numérique des fibres optiques du bundle, assurant ainsi un éclairage parfaitement homogène des fibres optiques du bundle.

La figure 5 représente un second mode de réalisation de ces moyens de conjugaison. Sur cette vue, seule une sortie de source fibrée est représentée. Comme on le verra sur la figure 8, il est facile d'adapter ce dispositif à la mise en oeuvre de deux sources fibrées. Les moyens de conjugaison comportent un système afocal optique comprenant une première lentille L1 et une seconde lentille L2, ledit système disposé entre l'extrémité de sortie de la de la source fibrée SF et les extrémités des fibres optiques de l'entrée du bundle EB, le foyer principal image de la première lentille L1 étant confondu avec le foyer principal objet de la seconde lentille L2. L'extrémité de sortie de la source fibrée SF étant disposée sensiblement sur l'axe optique xx et au foyer principal objet de ladite première lentille L1 et les premières extrémités des fibres optiques de l'entrée du bundle étant disposées sensiblement sur l'axe optique xx et au foyer principal image de la seconde lentille L2.

Un diffuseur optique D ayant la forme d'une lame mince est disposé au voisinage du foyer principal image de la première lentille L1 et au foyer principal objet de la seconde lentille L2 comme on le voit sur la figure 5.

Le système afocal peut avoir un grandissement unitaire. De préférence, l'ouverture numérique des lentilles constituant le système afocal est sensiblement égale à l'ouverture numérique des fibres optiques du bundle. En outre, de préférence, l'ouverture numérique des fibres optiques constituant le bundle est sensiblement égale à l'ouverture numérique de la première fibre optique. Lorsque ces trois conditions sont réunies, les distances focales F1 de la lentille L1 et F2 de la lentille L2 sont égales. Cette disposition permet d'obtenir à la fois un éclairement homogène au niveau des fibres optiques du bundle et un angle d'ouverture des faisceaux d'éclairement parfaitement adapté à l'ouverture numérique des fibres optiques du bundle. Cependant, il est très facile d'adapter cette configuration à des surfaces de fibres plus importantes ou à des ouvertures numériques des fibres optiques différentes en changeant soit les focales des lentilles, soit l'angle de diffusion du diffuseur.

On peut également, comme illustré sur la figure 6, adapter parfaitement la surface éclairée du bundle en le translatant sur l'axe optique du système afocal. Ainsi, une translation d'une valeur de d permet de passer d'une surface éclairée de bundle de diamètre $\phi1$ à une surface éclairée de bundle de diamètre $\phi2$. Cette distance d correspond à une distance d'ajustement autour de la distance focale de la deuxième lentille L2. Cette distance d'ajustement dépend de l'ouverture numérique des fibres optiques constituant le bundle, du diamètre $\phi2$ du bundle et du diamètre $\phi1$ de la première fibre optique. Lorsque le diamètre $\phi2$ est égal au diamètre $\phi1$, c'est-à-dire lorsque le diamètre de la première fibre optique est égal au diamètre de la surface d'entrée EB du bundle, il n'y a pas d'ajustement à prévoir. L'entrée du bundle EB est disposée à la distance focale F2 de la lentille L2. Cependant, il est usuel que le diamètre $\phi2$ du bundle EB soit différent, et en général supérieur, au diamètre $\phi1$ de la première fibre optique. Par exemple, on peut avoir $\phi2$ égal à 2000 $\mu$m tandis que $\phi1$ est égal à 1500 $\mu$m.

Dans ce cas, la distance d'ajustement est obtenue par l'expression :

$$d = (\phi2 - \phi1) / \tan(\alpha)$$

avec ON = $\sin \alpha$, ON représentant l'ouverture numérique des fibres optiques du bundle.

En considérant une ouverture ON de 0,39, un diamètre $\phi2$ égal à 2000 $\mu$m et un diamètre $\phi1$ égal à 1500 $\mu$m, d est égal à 1180 $\mu$m. Ainsi, la distance d'ajustement est de l'ordre du millimètre.

On comprend alors que la surface d'entrée du bundle est placée sensiblement à la distance focale F2 de la lentille L2, c'est-à-dire à la distance focale F2 éventuellement corrigée de la distance d'ajustement d.

Par rapport au premier mode de réalisation, ce deuxième mode de réalisation, utilisant un système optique afocal, est avantageux car il permet de placer le bundle à une distance plus importante de la première fibre optique. Cela permet, par exemple, de placer plus aisément une lame dichroïque entre la première fibre optique et le bundle, dans le cas ou plusieurs sources d'éclairage sont mises en oeuvre.

En effet, dans un certain nombre d'applications, il est nécessaire que le dispositif d'éclairage comporte deux sources d'éclairage différentes qui sont généralement une source de lumière blanche et une source infrarouge permettant de créer une lumière de fluorescence. Différents moyens optiques permettent de coupler les deux sources aux moyens de conjugaison optique de façon que les fibres optiques du bundle soient éclairées simultanément par les rayonnements issus des deux sources.

Dans un premier mode de réalisation représenté sur la figure 7, le dispositif d'éclairage comporte un coupleur Y à fibre optique comprenant deux entrées et une sortie, la première source de lumière S1 étant disposée devant la première entrée du coupleur, la seconde source de lumière S2 étant disposée devant la seconde entrée du coupleur, la sortie du coupleur étant disposée au foyer principal objet de la première lentille. Le coupleur a deux inconvénients. D'une part, les coupleurs standards ont des diamètres d'entrée de taille limitée. Généralement, le diamètre ne dépasse pas le millimètre. Ce faible diamètre peut poser des problèmes de couplage dans des bundles comportant un grand nombre de fibres optiques et donc de surface d'entrée de dimension nettement plus importante. Les diamètres des fibres optiques des bundles font typiquement quelques centaines de microns. D'autre part, par nature, le coupleur optique a une transmission sur chaque voie qui n'excède pas 60%.

Aussi, dans un second mode de réalisation représenté en figure 8, le mélange des deux sources fibrées est assurée par une lame semi-réfléchissante LSR disposée à 45 degrés sur l'axe optique des moyens de conjugaison après la lentille L1. Ces moyens de conjugaison comportent une troisième lentille L3 dont l'axe optique zz est disposé perpendiculairement au premier axe optique xx et passe par le centre de la lame semi-réfléchissante. Cette troisième lentille a une focale F3. La seconde source fibrée SF2 est disposée au foyer de cette lentille L3. L'ensemble optique constitué de la lentille L3, de la lame semi-réfléchissante LSR et de la lentille L2 constitue un second afocal identique au premier. Cette disposition a deux avantages sur la précédente. D'une part, il est plus facile d'adapter les diamètres des sources fibrées à celui de l'entrée du bundle. D'autre part, il est possible d'utiliser une lame dichroïque dont les coefficients de transmission et de réflexion sont optimisés de façon parfaitement transmettre ou réfléchir les bandes spectrales des deux sources.

Ce mode de réalisation, mettant en oeuvre deux sources de lumières, permet d'utiliser un unique bundle, chaque fibre du bundle pouvant être éclairée soit par la première source de lumière, soit par la deuxième source de lumière, soit par les deux sources de lumières simultanément.

Le dispositif d'éclairage selon l'invention peut être monté dans différents dispositifs nécessitant un éclairage parfaitement homogène de faible dimension. Ainsi, on peut l'utiliser dans des sondes peropératoires comprenant une caméra de faible dimension. Les deuxièmes extrémités des fibres optiques du bundle entourent alors la tête optique de la caméra. L'anneau d'éclairage a une épaisseur qui ne dépasse pas le millimètre et il peut parfaitement remplir les conditions de sécurité oculaire dans la mesure où l'éclairement laser est réparti sur un grand nombre de fibres optiques, chaque fibre délivrant une puissance lumineuse homogène.

## Revendications

1. Dispositif d'éclairage comprenant au moins une source de lumière fibrée (SF) et un transport de lumière (OFB) comportant une pluralité de secondes fibres optiques multimodes (FO), la lumière issue de la source de lumière étant conduite à une extrémité de sortie d'une première fibre optique, les premières extrémités des secondes fibres optiques étant regroupées dans une première surface (EB), les secondes extrémités des fibres optiques étant disposées sur la périphérie d'une seconde surface (SB), **caractérisé en ce que** le dispositif d'éclairage comporte :

   Un système afocal optique comprenant une première lentille (L1) et une seconde lentille (L2), ledit système disposé entre l'extrémité de sortie de la première fibre optique et les premières extrémités des secondes fibres optiques, le foyer principal image de la première lentille étant confondu avec le foyer principal objet de la seconde lentille ;
   L'extrémité de sortie de la première fibre optique étant disposée sensiblement sur l'axe optique et au foyer principal objet de ladite première lentille ; et lesdites premières extrémités des secondes fibres optiques étant disposées sensiblement sur l'axe optique et sensiblement au foyer principal image de ladite seconde lentille ; un diffuseur optique (D) ayant la forme d'une lame disposé au voisinage du foyer principal image de la première lentille et au foyer principal objet de la seconde lentille.

2. Dispositif d'éclairage selon la revendication 1, **caractérisé en ce que** le système afocal a un grandissement unitaire.

3. Dispositif d'éclairage selon la revendication 1, **ca-**

**ractérisé en ce que** le système afocal a une ouverture numérique sensiblement égale à l'ouverture numérique des secondes fibres optiques.

4. Dispositif d'éclairage selon la revendication 1, **caractérisé en ce que** les premières extrémités des secondes fibres optiques sont translatées par rapport au foyer principal image de ladite seconde lentille, selon une distance d'ajustement d, déterminée en fonction de la surface de ladite première fibre optique et de la surface de la première surface (EB).

5. Dispositif d'éclairage (E) comprenant au moins une source de lumière fibrée (SF) et un transport de lumière (OFB) comportant une pluralité de secondes fibres optiques multimodes (FO), la lumière issue de la source de lumière étant conduite à une extrémité de sortie d'une première fibre optique, les premières extrémités des secondes fibres optiques étant regroupées dans une première surface (EB), les secondes extrémités des fibres optiques étant disposées sur la périphérie d'une seconde surface (SB), **caractérisé en ce que** le dispositif d'éclairage comporte :

    une première lentille (L1) disposée entre l'extrémité de sortie de la première fibre optique et les premières extrémités des secondes fibres optiques, ladite extrémité de sortie de la première fibre optique étant disposée sensiblement sur l'axe optique et au foyer principal objet de ladite première lentille ; et lesdites premières extrémités des secondes fibres optiques étant disposées sensiblement sur l'axe optique et au foyer principal image de ladite première lentille ;
    un diffuseur optique (D) ayant la forme d'une lame disposé entre la première lentille et les premières extrémités des secondes fibres optiques.

6. Dispositif d'éclairage selon la revendication 5, **caractérisé en ce que** la focale de la première lentille est sensiblement égale au quotient du rayon de la première surface circulaire par l'ouverture numérique de la première fibre optique.

7. Dispositif d'éclairage selon la revendication 5, **caractérisé en ce que** l'angle de diffusion du diffuseur optique est sensiblement égal à l'ouverture numérique des secondes fibres optiques.

8. Dispositif d'éclairage selon l'une des revendications précédentes, **caractérisé en ce que** le dispositif d'éclairage comporte une seconde source de lumière (S2) et un coupleur optique (Y) comprenant deux entrées et une sortie, la seconde source ayant un second spectre d'émission différent du premier spectre d'émission de la première source (S1), la première source de lumière étant disposée devant la première entrée du coupleur, la seconde source de lumière étant disposée devant la seconde entrée du coupleur, la sortie du coupleur étant disposée au foyer principal objet de la première lentille.

9. Dispositif d'éclairage selon l'une des revendications 1 à 7, **caractérisé en ce que** le dispositif d'éclairage comporte une seconde source de lumière fibrée (SF2) et une lame semi-réfléchissante (LSR), la seconde source ayant un second spectre d'émission différent du premier spectre d'émission de la première source, la lumière issue de la seconde source de lumière étant conduite à une extrémité de sortie d'une troisième fibre optique, la lame semi-réfléchissante comportant un traitement dichroïque optimisé de façon à avoir un maximum de transmission du premier spectre d'émission et un maximum de réflexion du second spectre d'émission, la lame semi-réfléchissante étant disposée de façon que l'image de l'extrémité de sortie de la troisième fibre optique par réflexion sur la lame semi-réfléchissante se superpose à l'image par transmission de l'extrémité de sortie de la première fibre optique.

10. Sonde peropératoire comprenant une caméra et un dispositif d'éclairage, **caractérisé en ce que** le dispositif d'éclairage est selon l'une des revendications 8 ou 9, le premier spectre de la première source étant situé dans le visible, le second spectre de la seconde source étant situé dans un spectre de fluorescence situé dans le proche infrarouge, les secondes extrémités des fibres optiques étant disposées de façon sensiblement équidistante sur la périphérie de l'objectif de la caméra.

**Patentansprüche**

1. Beleuchtungsvorrichtung, die wenigstens eine Lichtfaserquelle (SF) und einen Lichttransport (OFB) umfasst, der mehrere zweite Multimoden-Lichtleitfasern (FO) umfasst, wobei das von der Lichtquelle ausgehende Licht zu einem Ausgangsende einer ersten Lichtleitfaser geführt wird, wobei die ersten Enden der zweiten Lichtleitfasern zu einer ersten Oberfläche (EB) gruppiert werden, wobei die zweiten Enden der Lichtleitfasern auf der Peripherie einer zweiten Oberfläche (SB) angeordnet sind, **dadurch gekennzeichnet, dass** die Beleuchtungsvorrichtung Folgendes umfasst:

    ein afokales optisches System, das eine erste Linse (L1) und eine zweite Linse (L2) umfasst, wobei das System zwischen dem Ausgangsende der ersten Lichtleitfaser und den ersten Enden der zweiten Lichtleitfasern angeordnet ist, wobei der Bildhauptfokus der ersten Linse mit dem Objekthauptfokus der zweiten Linse zusammenfällt;

wobei das Ausgangsende der ersten Lichtleitfaser im Wesentlichen auf der optischen Achse und auf dem Objekthauptfokus der ersten Linse angeordnet ist; und wobei die ersten Enden der zweiten Lichtleitfasern im Wesentlichen auf der optischen Achse und im Wesentlichen auf dem Bildhauptfokus der zweiten Linse angeordnet sind;

einen optischen Diffusor (D) in Form einer Platte, die in der Nähe des Bildhauptfokus der ersten Linse und auf dem Objekthauptfokus der zweiten Linse angeordnet ist.

2. Beleuchtungsvorrichtung nach Anspruch 1, **dadurch gekennzeichnet, dass** das afokale System eine Vergrößerung von 1 hat.

3. Beleuchtungsvorrichtung nach Anspruch 1, **dadurch gekennzeichnet, dass** das afokale System eine numerische Apertur im Wesentlichen gleich der numerischen Apertur der zweiten Lichtleitfasern hat.

4. Beleuchtungsvorrichtung nach Anspruch 1, **dadurch gekennzeichnet, dass** die ersten Enden der zweiten Lichtleitfasern mit Bezug auf den Bildhauptfokus der zweiten Linse gemäß einer Justagedistanz d verschoben sind, bestimmt in Abhängigkeit von der Fläche der ersten Lichtleitfaser und der Fläche der ersten Oberfläche (EB).

5. Beleuchtungsvorrichtung (E), die wenigstens eine Lichtfaserquelle (SF) und einen Lichttransport (OFD) umfasst, der mehrere zweite Multimoden-Lichtleitfasern (FO) umfasst, wobei das aus der Lichtquelle austretende Licht zu einem Ausgangsende einer ersten Lichtleitfaser geleitet wird, wobei die ersten Enden der zweiten Lichtleitfasern in einer ersten Oberfläche (EB) gruppiert werden, wobei die zweiten Enden der Lichtleitfasern auf der Peripherie einer zweiten Oberfläche (SB) angeordnet sind, **dadurch gekennzeichnet, dass** die Beleuchtungsvorrichtung Folgendes umfasst:

eine erste Linse (L1), die zwischen dem Ausgangsende der ersten Lichtleitfaser und den ersten Enden der zweiten Lichtleitfasern angeordnet ist, wobei das Ausgangsende der ersten Lichtleitfaser im Wesentlichen auf der optischen Achse und auf dem Objekthauptfokus der ersten Linse angeordnet ist; und wobei die ersten Enden der zweiten Lichtleitfasern im Wesentlichen auf der optischen Achse und auf dem Bildhauptfokus der ersten Linse angeordnet sind;

einen optischen Diffusor (D) mit der Form einer Platte, die zwischen der ersten Linse und den ersten Enden der zweiten Lichtleitfasern angeordnet ist.

6. Beleuchtungsvorrichtung nach Anspruch 5, **dadurch gekennzeichnet, dass** der Fokus der ersten Linse im Wesentlichen gleich dem Quotienten des Radius der ersten kreisförmigen Oberfläche durch die numerische Apertur der ersten Lichtleitfaser ist.

7. Beleuchtungsvorrichtung nach Anspruch 5, **dadurch gekennzeichnet, dass** der Diffusionswinkel des optischen Diffusors im Wesentlichen gleich der numerischen Apertur der zweiten Lichtleitfasern ist.

8. Beleuchtungsvorrichtung nach einem der vorherigen Ansprüche, **dadurch gekennzeichnet, dass** die Beleuchtungsvorrichtung eine zweite Lichtquelle (S2) und einen Opto-Koppler (Y) umfasst, der zwei Eingänge und einen Ausgang umfasst, wobei die zweite Quelle ein zweites Emissionsspektrum hat, das sich vom ersten Emissionsspektrum der ersten Quelle (S1) unterscheidet, wobei die erste Lichtquelle vor dem ersten Eingang des Kopplers angeordnet ist, wobei die zweite Lichtquelle vor dem zweiten Eingang des Kopplers angeordnet ist, wobei der Ausgang des Kopplers auf dem Objekthauptfokus der ersten Linse angeordnet ist.

9. Beleuchtungsvorrichtung nach einem der Ansprüche 1 bis 7, **dadurch gekennzeichnet, dass** die Beleuchtungsvorrichtung eine zweite Lichtfaserquelle (SF2) und eine halbreflektierende Platte (LSR) umfasst,

wobei die zweite Quelle ein zweites Emissionsspektrum hat, das sich vom ersten Emissionsspektrum der ersten Quelle unterscheidet, wobei das von der zweiten Lichtquelle austretende Licht zu einem Ausgangsende einer dritten Lichtleitfaser geleitet wird, wobei die teilreflektierende Platte eine optimierte dichroitische Verarbeitung hat, so dass sie eine maximale Transmission des ersten Emissionsspektrums und eine maximale Reflexion des zweiten Emissionsspektrums hat, wobei die halbreflektierende Platte so angeordnet ist, dass das Bild des Ausgangsendes der dritten Lichtleitfaser durch Reflexion auf der halbreflektierenden Platte das Bild durch Transmission des Ausgangsendes der ersten Lichtleitfaser überlagert.

10. Peroperative Sonde, die eine Kamera und eine Beleuchtungsvorrichtung umfasst, **dadurch gekennzeichnet, dass** die Beleuchtungsvorrichtung gemäß Anspruch 8 oder 9 ist, wobei sich das erste Spektrum der ersten Quelle im sichtbaren Bereich befindet, das zweite Spektrum der zweiten Quelle sich in einem Fluoreszenzspektrum befindet, das sich im Nah-Infrarot-Bereich befindet, wobei die zweiten Enden der Lichtleitfasern auf im Wesentlichen äquidistante Weise auf der Peripherie des Objektivs der Kamera angeordnet sind.

**Claims**

1. Lighting device comprising at least one fibre light source (SF) and a light transport (OFB) comprising a plurality of second multimode optical fibres (FO), the light from the light source being guided to an output end of a first optical fibre, the first ends of the second optical fibres being grouped within a first surface (EB), the second ends of the optical fibres being arranged over the periphery of a second surface (SB), **characterised in that** the lighting device comprises:

   an afocal optical system comprising a first lens (L1) and a second lens (L2), the system being arranged between the output end of the first optical fibre and the first ends of the second optical fibres, the main image focal point of the first lens coinciding with the main object focal point of the second lens;
   the output end of the first optical fibre being arranged substantially on the optical axis and at the main object focal point of the first lens; and the first ends of the second optical fibres being arranged substantially on the optical axis and substantially at the main image focal point of the second lens;
   an optical diffuser (D) being in the form of a plate arranged in the vicinity of the main image focal point of the first lens and at the main object focal point of the second lens.

2. Lighting device according to claim 1, **characterised in that** the afocal system has a unitary magnification.

3. Lighting device according to claim 1, **characterised in that** the afocal system has a numerical aperture substantially equal to the numerical aperture of the second optical fibres.

4. Lighting device according to claim 1, **characterised in that** the first ends of the second optical fibres are translated with respect to the main image focal point of the second lens in accordance with an adjustment distance d determined as a function of the surface-area of the first optical fibre and of the surface-area of the first surface (EB).

5. Lighting device (E) comprising at least one fibre light source (SF) and a light transport (OFB) comprising a plurality of second multimode optical fibres (FO), the light from the light source being guided to an output end of a first optical fibre, the first ends of the second optical fibres being grouped within a first surface (EB), the second ends of the optical fibres being arranged over the periphery of a second surface (SB), **characterised in that** the lighting device comprises:

   a first lens (L1) arranged between the output end of the first optical fibre and the first ends of the second optical fibres, the output end of the first optical fibre being arranged substantially on the optical axis and at the main object focal point of the first lens; and the first ends of the second optical fibres being arranged substantially on the optical axis and at the main image focal point of the first lens;
   an optical diffuser (D) being in the form of a plate arranged between the first lens and the first ends of the second optical fibres.

6. Lighting device according to claim 5, **characterised in that** the focal length of the first lens is substantially equal to the quotient of the radius of the first circular surface divided by the numerical aperture of the first optical fibre.

7. Lighting device according to claim 5, **characterised in that** the scattering angle of the optical diffuser is substantially equal to the numerical aperture of the second optical fibres.

8. Lighting device according to any one of the preceding claims, **characterised in that** the lighting device comprises a second light source (S2) and an optical coupler (Y) comprising two inputs and one output, the second source having a second emission spectrum different from the first emission spectrum of the first source (S1), the first light source being arranged in front of the first input of the coupler, the second light source being arranged in front of the second input of the coupler, the output of the coupler being arranged at the main object focal point of the first lens.

9. Lighting device according to any one of claims 1 to 7, **characterised in that** the lighting device comprises a second fibre light source (SF2) and a semi-reflective plate (LSR),
   the second source having a second emission spectrum different from the first emission spectrum of the first source, the light from the second light source being guided to an output end of a third optical fibre, the semi-reflective plate comprising a dichroic coating optimised so as to have a transmission maximum of the first emission spectrum and a reflection maximum of the second emission spectrum, the semi-reflective plate being arranged in such a manner that the image of the output end of the third optical fibre, by reflection on the semi-reflective plate, is superimposed onto the image by transmission of the output end of the first optical fibre.

10. Intraoperative probe comprising a camera and a lighting device, **characterised in that** the lighting device is in accordance with either claim 8 or claim 9, the first spectrum of the first source being located in the visible wavelength, the second spectrum of the second source being located in a fluorescence spectrum in the near-infrared wavelength, the second ends of the optical fibres being arranged so as to be substantially equidistant over the periphery of the objective lens of the camera.

## FIG. 1

## FIG. 2

Entrée du bundle          Sortie du bundle

## FIG. 3

FIG. 4

FIG. 5

FIG. 6

FIG. 7

FIG. 8

**RÉFÉRENCES CITÉES DANS LA DESCRIPTION**

**Documents brevets cités dans la description**

- FR 2010750
- US 4272156 A
- US 5412749 A
- US 4964692 A
- WO 9805987 A
- US 20040156098 A1